**Europäisches Patentamt**

**European Patent Office**

**Office européen des brevets**

(19)

(11) Veröffentlichungsnummer : **0 032 524**
**B1**

(12)

# EUROPÄISCHE PATENTSCHRIFT

(45) Veröffentlichungstag der Patentschrift :
**22.06.83**

(21) Anmeldenummer : **80106241.5**

(22) Anmeldetag : **15.10.80**

(51) Int. Cl.³ : **B 01 J 23/94**, C 07 C 51/12,
C 07 C 51/48

(54) **Verfahren zur extraktiven Aufarbeitung der bei der Hydrocarboxylierung eingesetzten kobalthaltigen Katalysatoren.**

(30) Priorität : **12.12.79 DE 2949939**

(43) Veröffentlichungstag der Anmeldung :
**29.07.81 Patentblatt 81/30**

(45) Bekanntmachung des Hinweises auf die Patenterteilung : **22.06.83 Patentblatt 83/25**

(84) Benannte Vertragsstaaten :
**BE FR GB IT NL**

(56) Entgegenhaltungen :
**DE A 2 447 068**
**DE A 2 630 328**
**DE B 1 150 065**
**DE B 1 245 918**
**DE B 2 159 139**
**US A 3 507 891**

(73) Patentinhaber : **CHEMISCHE WERKE HÜLS AG**
**Postfach 1320**
**D-4370 Marl 1 (DE)**

(72) Erfinder : **Hofmann, Peter, Dr.**
**Lipper Weg 193**
**D-4370 Marl (DE)**

EP 0 032 524 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur extraktiven Aufarbeitung der bei der Hydrocarboxylierung eingesetzten kobalthaltigen Katalysatoren

Es ist bekannt, daß man durch Umsetzung von Olefinen mit Kohlenmonoxid und einer H-aciden Komponente, wie Wasser oder Alkanol, in Gegenwart eines Katalysators, der ein Metall der 8. Nebengruppe des Periodischen Systems der Elemente und gegebenenfalls einen Promotor enthält, Fettsäuren bzw. die entsprechenden Fettsäurederivate herstellen kann (J. Falbe, Synthesen mit Kohlenmonoxid, Springer-Verlag, Berlin, Heidelberg, New York, 1967).

Als besonders bevorzugte Variante dieser als Hydrocarboxylierung bezeichneten Reaktion hat sich die Umsetzung in Gegenwart von kobalthaltigen Katalysatoren erwiesen. Eine besonders bevorzugte Ausführungsform besteht darin, daß man zusätzlich einen Promotor, und zwar insbesondere Pyridin oder ein nicht-orthosubstituiertes Alkylpyridin zusetzt.

Ein wesentliches Problem dieser homogenen katalysierten Reaktion ist die Rückgewinnung des relativ teuren Kobalts aus dem Reaktionsgemisch in einer Form, die seinen Wiedereinsatz als Katalysator gestattet.

Ein in der DE-AS 21 59 139 beschriebenes Verfahren löst dieses Problem dadurch, daß die Umsetzung des Olefins mit Kohlenmonoxid in Gegenwart eines Überschusses an Alkanol und Paraffin durchgeführt wird bzw. Paraffin nach beendeter Umsetzung zugegeben wird. Auf diese Weise bildet sich ein zweiphasiges Gemisch. Die untere Phase, die überwiegend aus Alkanol und Promotor besteht, enthält maximal ca. 97 % des als Katalysator eingesetzten Kobalts. Die obere paraffinische Phase wird im wesentlichen aus unumgesetztem Olefin und Reaktionsprodukten gebildet.

Die untere, den Katalysator noch in aktiver Form enthaltende Phase kann erneut in die Reaktion eingesetzt werden. Der sich hieraus ergebende Vorteil wird jedoch durch den Verlust von ca. 3 % des eingesetzten Kobalts mehr als aufgewogen. Ein Hydrocarboxylierungsverfahren kann nämlich nur dann als wirtschaftlich zufriedenstellend betrachtet werden, wenn auch das in der paraffinischen Phase enthaltende Kobalt zurückgewonnen wird. Angesichts des für das Verfahren erforderlichen Alkanolüberschusses und Paraffinzusatzes würde sich eine solche Aufarbeitung allerdings sehr aufwendig gestalten.

Ein weiteres Verfahren zur Rückgewinnung des Kobalts ist in der US-PS 3 507 891 beschrieben. Das Verfahren ist dadurch gekennzeichnet, daß das Kobalt zusammen mit dem Sumpf der destillativen Aufarbeitung des Reaktionsgemisches wiedergewonnen wird.

Wird dabei das Reaktionsgemisch vor der destillativen Aufarbeitung einer oxidativen Behandlung, z. B. mit Luft, unterzogen, so wird der Katalysator in einer Form zurückgewonnen, aus der sich erst wieder unter den Bedingungen der Hydrocarboxylierung die aktive Katalysatorspecies zurückbildet. Nur beim Einsatz von Alkylpyridinen als Promotoren soll auf eine oxidative Behandlung des Reaktionsgemisches verzichtet werden können. In Gegenwart dieser Promotoren sollen sich nämlich unter den Destillationsbedingungen thermostabile Komplexe bilden, die ihre Aktivität beibehalten.

Das Verfahren gemäß der US-PS 3 507 891 bietet zwar eine Möglichkeit zur weitgehend vollständigen Rückgewinnung des eingesetzten Kobalts, es zeigt aber ebenso wenig wie das Verfahren gemäß der DE-AS 21 59 139 einen Weg zur Abtrennung von hochsiedenden Substanzen und anderen störenden Verunreinigungen, die sich unvermeidlich bei jeder Hydrocarboxylierung als Nebenprodukt bilden.

Aufgabe der vorliegenden Erfindung war es daher, ein möglichst einfaches und weitgehend verlustfreies Verfahren zur Aufarbeitung des bei der Hydrocarboxylierung eingesetzten kobalthaltigen Katalysators zu entwickeln, das gleichzeitig die Abtrennung von hochsiedenden und unerwünschten Verunreinigungen gestattet.

Diese Aufgabe wurde dadurch gelöst, daß man das Reaktionsgemisch nach einer oxidativen Behandlung destilliert und den hierbei anfallenden kobalthaltigen Destillationssumpf mit einer 1 bis 4 Kohlenstoffatome enthaltenden Carbonsäure und Wasser behandelt, die entstehenden Phasen trennt und aus der Carbonsäure-Wasser-Phase das Kobalt in Form eines carbonsauren Salzes zurückgewinnt.

Das erfindungsgemäße Verfahren kann im Prinzip bei allen Hydrocarboxylierungsreaktionen, die in Gegenwart eines kobalthaltigen katalysators durchgeführt werden, angewandt werden (z. B. Verfahren gemäß US-PS 3 507 891 und deutscher Patentanmeldung P 29 12 489.8). So ist vor allem die Wahl des eingesetzten Olefins unkritisch, d. h. es können sowohl geradkettige oder verzweigte α-Olefine als auch Olefine mit innenständiger Doppelbindung eingesetzt werden. Aber auch Olefine mit mehr als einer Doppelbindung und solche mit Substituenten, z. B. Aryl-, Cyano-, Carboximethyl- und Hydroxylgruppen, sind geeignet.

Im allgemeinen werden Olefine mit 2 bis 40, vorzugsweise mit 4 bis 20 Kohlenstoffatomen, eingesetzt, die nach bekannten Verfahren des Standes der Technik erhalten werden können. So können z. B. α-Olefine durch die Aufbaureaktion von Ethylen nach Ziegler oder durch Wachscracken, Olefine mit innenständiger Doppelbindung durch Dehydrierung oder Chlorierung und anschließender Dehydrochlorierung von Paraffinen gewonnen werden.

Bei dem zuletzt genannten Verfahren werden in der Regel Paraffinschnitte, d. h. Gemische unterschiedlicher C-Zahl eingesetzt, so daß auch die erhaltenen Olefine keine einheitliche C-Zahl aufweisen. Außerdem kommen in diesen Olefin-Gemischen natürlich alle denkbaren isomeren Formen vor.

Neben den reinen — gegebenenfalls substituierten — Olefinen können auch solche mit einem Gehalt an Paraffinen eingesetzt werden. Der Paraffingehalt rührt daher, daß bei der Olefinherstellung keine vollständigen Umsätze erreicht und die nicht umgesetzten Paraffine nicht oder nicht vollständig abgetrennt werden.

Nicht nur das eingesetzte Olefin, sondern auch die H-acide Verbindung, die mit dem Olefin und Kohlenmonoxid umgesetzt wird, ist für das erfindungsgemäße Verfahren unkritisch. Sowohl Wasser als auch Alkanole mit 1 bis 20, vorzugsweise 1 bis 4 Kohlenstoffatomen, können eingesetzt werden.

Weiterhin ist es unwesentlich, welche Kobaltverbindung bei der Hydrocarboxylierung verwendet wird. Carbonyle des Kobalts sind ebenso geeignet wie carbonsaure Kobaltsalze bzw. Salze des Kobalts mit anorganischen Säuren. Vorzugsweise kommen solche carbonsauren Kobaltsalze zum Einsatz, deren Anionen in Form der entsprechenden Carbonsäuren bzw. Carbonsäureester bei der Hydrocarboxylierung gebildet werden.

Neben der Kobaltverbindung wird ein sogenannter Promotor eingesetzt. Hierfür kommen Pyridin, alle nicht orthosubstituierten Alkylpyridine und N-Methyl-pyrrolidon infrage.

Schließlich sind die Reaktionsbedingungen, unter denen die Hydrocarboxylierung durchgeführt wird, für das erfindungsgemäße Verfahren nicht von Bedeutung. Im allgemeinen werden Hydrocarboxylierungsverfahren bei Temperaturen von 80 bis 300, vorzugsweise 150 bis 220 °C, und Kohlenmonoxiddrucken von 10 bis 800, vorzugsweise 100 bis 300 bar, durchgeführt.

Verfahrenskritisch für das vorliegende Verfahren ist jedoch die oxidative Behandlung des Reaktionsgemisches vor der Rückgewinnung des Kobalts mit Sauerstoff oder einem sauerstoffhaltigen Gas, vorzugsweise Luft, bei Temperaturen von 20 bis 150, vorzugsweise 70 bis 120 °C. Diese Behandlung, die bereits in der US-PS 3 507 891, Spalte 4, Zeilen 21 bis 43, und in der nicht zum Stand der Technik gehörenden deutschen Patentanmeldung P 29 12 489.8, Seite 5, Absatz 2, beschrieben ist, wird solange durchgeführt, bis die bei der folgenden destillativen Aufarbeitung zur Abscheidung von metallischem Kobalt führenden Kobaltverbindungen oxidativ zerstört sind.

Bei der sich anschließenden destillativen Aufarbeitung werden die flüchtigen Anteile des Reaktionsgemisches entweder in einem Schritt oder stufenweise bei Sumpftemperaturen bis maximal 350 °C abgetrennt. Der Kobaltgehalt des hierbei anfallenden Destillationssumpfes soll 2 bis 30, vorzugsweise 4 bis 15 Gewichtsprozent, betragen.

Der kobalthaltige Rückstand kann entweder in seiner Gesamtheit oder teilweise erfindungsgemäß aufgearbeitet werden. Wählt man die Aufarbeitung nur eines Teils des kobalthaltigen Rückstandes, so richtet sich der Anteil des aufzuarbeitenden kobalthaltigen Rückstandes nach dem für die Hydrocarboxylierung angestrebten Niveau katalytischer Aktivität, der tolerierbaren Menge an Ballaststoffen, wie Hochsiedern, und dem für die Aufarbeitung erforderlichen Aufwand.

Für eine bequemere Handhabung des kobalthaltigen Rückstandes kann es zweckmäßig sein, diesen mit einem oder mehreren Lösemitteln zu verdünnen. Als Lösemittel kommen solche infrage, die bei der anschließenden Carbonsäure/Wasser-Behandlung nicht die Ausbildung zweier separater Phasen behindern, z. B. Paraffine, vorzugsweise solche mit 5 bis 10 Kohlenstoffatomen, oder Aromaten, vorzugsweise Benzol, Toluol oder Xylol.

Zur Behandlung des gegebenenfalls mit Lösemitteln verdünnten kobalthaltigen Rückstandes sind Carbonsäuren mit 1 bis 4 Kohlenstoffatomen geeignet. Vorzugsweise werden Essigsäure und/oder Propionsäure eingesetzt. Pro Grammatom sind mindestens 2 Mol Carbonsäure erforderlich. Im allgemeinen arbeitet man mit 2 bis 250, vorzugsweise 2 bis 100 Mol Carbonsäure pro Grammatom Kobalt.

Es ist für das erfindungsgemäße Verfahren unkritisch, ob das zur Behandlung und Phasentrennung erforderliche Wasser zusammen mit der Carbonsäure oder erst anschließend zugegeben wird.

Das Wasser wird in einer Menge zugegeben, die ausreichend ist, eine Trennung in organische obere Phase und eine das Kobalt in gelöster Form enthaltende untere wäßrig-carbonsaure Phase zu bewirken. Die Wassermenge beträgt im allgemeinen die 0,1- bis 10fache Gewichtsmenge der Carbonsäure.

Die Behandlung des kobalthaltigen Katalysatorrückstandes kann in allen dafür geeigneten Apparaturen, wie Rührkessel, Rührkesselkaskade und Gegenstromextraktor, erfolgen.

Die Behandlungstemperatur soll im allgemeinen den Siedepunkt der tiefstsiedenden Komponente des 2phasigen Gemisches nicht übersteigen. Vorzugsweise liegt sie im Bereich von 20 bis 100 °C.

Nach der Behandlung mit Carbonsäure und Wasser erfolgt — gegebenenfalls nach einer gewissen Wartezeit — die Trennung der gebildeten Phasen in einer dafür geeigneten Vorrichtung, z. B. Scheidetrichter oder Absetzbehälter. Dieser separate Trennschritt kann entfallen, wenn bereits während der Behandlung eine ausreichende Phasentrennung eintritt, z. B. im Gegenstromextraktor.

Sowohl die Behandlung des kobalthaltigen Katalysatorrückstandes mit Carbonsäure und Wasser als auch die gegebenenfalls erforderliche anschließende Phasentrennung können zur Erhöhung der Kobaltrückgewinnung beliebig oft diskontinuierlich oder kontinuierlich wiederholt werden.

Die gegebenenfalls vereinten kobalthaltigen Phasen werden anschließend dadurch aufgearbeitet, daß Wasser und überschüssige Carbonsäure, z. B. durch Destillation, abgetrennt werden. Zurück bleibt das carbonsaure Kobaltsalz.

Sofern dieses carbonsaure Kobaltsalz sich nicht in einem der zur Hydrocarboxylierung eingesetzten Reaktanten löst und in Form dieser Lösung problemlos in den Hydrocarboxylierungsprozeß zurückgeführt werden kann, ist als letzter Schritt seine Umwandlung erforderlich. Diese besteht in einer Umsetzung mit einer solchen Carbonsäure, deren Kobaltsalz dann in mindestens einem der Reaktanten löslich ist. Zum Beispiel kann Kobaltacetat, das sich nicht in höheren Alkanolen, Olefinen und den gegebenenfalls eingesetzten Promotoren löst, mit Hilfe von z. B. 2-Ethylhexansäure in das sogenannte Kobaltoctoat überführt werden, das in Alkanolen mit einer C-Zahl von ≥ 2 löslich ist. Die bei der obengenannten Umsetzung freiwerdende Essigsäure kann destillativ abgetrennt und in den Aufarbeitungsprozeß zurückgeführt werden.

Die mehr oder weniger vom Kobalt befreite organische Phase wird entweder verworfen oder, sofern es die Wirtschaftlichkeit des Verfahrens erfordert, aufgearbeitet. Dies kann z. B. durch Destillation geschehen. Das gegebenenfalls zur Verdünnung des kobalthaltigen Rückstandes zugesetzte Lösemittel kann hierbei wiedergewonnen und die auf dieser Stufe anfallenden Carbonsäuren können z. B. wieder an geeigneter Stelle des erfindungsgemäßen Verfahrens eingesetzt werden.

Wie bereits dargelegt, kann das erfindungsgemäße Verfahren bei allen Hydrocarboxylierungsprozessen mit Erfolg eingesetzt werden, bei denen ein kobalthaltiger Katalysator verwendet wird.

Alle Prozentangaben — auch in den nachfolgenden Beispielen, die das erfindungsgemäße Verfahren erläutern — sind, sofern nicht anders angegeben, Gewichtsprozente.

Beispiel 1

Hydrocarboxylierung :

In einem 5 1-VA-Rührautoklaven werden 1 680 g eines statistischen Isomerengemisches linearer Dodecene mit innenständiger Doppelbindung (1-Dodecen-Anteil < 1 %), 800 g Methanol, 167 g eines 10 %igen Co-Tridecanoats und 279 g γ-Picolin bei 180 °C mit CO mit einem $H_2$-Gehalt von 2 Vol.-% bei 200 bar Warmdruck umgesetzt. Nach 3 Stunden wird die Reaktion bei einem Olefinumsatz von 87 % abgebrochen.

Oxidative Behandlung des Reaktionsaustrags :

Der gesamte Reaktionsaustrag von 3 126 g wird in einer mit Raschigringen gefüllten Rieselsäule (Länge : 1 m ; Innendurchmesser : 2,5 cm) bei 80 °C im Gegenstrom mit 100 l Luft/h behandelt. Die Verweilzeit der von oben zulaufenden flüssigen Phase in der Rieselsäule beträgt 15 min.

Katalysatoraufarbeitung :

Aus dem so vorbehandelten Reaktionsaustrag werden durch stufenweise Destillation Methanol, γ-Picolin, unumgesetztes Dodecen und Tridecansäuremethylester abgetrennt. Nach dem Abtrennen der Esterfraktion verbleibt ein Rückstand von 198 g mit einem Kobaltgehalt von 8,43 %.

Der kobalthaltige Rückstand wird mit einer Mischung aus 400 g Essigsäure und 400 g Wasser 1 Stunde lang unter Rückfluß erhitzt. Die entstandenen Phasen werden in einem beheizbaren Scheidetrichter bei 90 °C getrennt. Man erhält eine obere organische Phase mit einem Gewicht von 188 g. Die untere wäßrig-essigsaure Phase wiegt 810 g und hat einen Kobaltgehalt von 2,04 %. Damit befinden sich 99,1 % des gesamten als Hydrocarboxylierungskatalysator eingesetzten Kobalts in der wäßrig-essigsauren Phase.

Die obere organische Phase wird nochmals mit 376 g eines Gemisches aus gleichen Gewichtsanteilen Essigsäure und Wasser versetzt, 15 min unter Rückfluß gekocht und die entstandenen Phasen in einem beheizbaren Scheidetrichter bei 90 °C getrennt. Die so erhaltene obere organische Phase wiegt 184 g. Die untere wäßrigessigsaure Phase wiegt 380 g und hat einen Kobaltgehalt von 0,037 %.

Die beiden durch Extraktion erhaltenen wäßrigessigsauren Phasen, die insgesamt 99,95 % des als Hydrocarboxylierungskatalysator eingesetzten Kobalts enthalten, werden vereinigt und anschließend am Rotationsverdampfer bei Wasserstrahlvakuum und einer Badtemperatur von 55 °C zur Trockne eingeengt. Man erhält 63,4 g eines violetten, kristallinen Feststoffs mit einem Kobaltgehalt von 26,3 %.

Wiedereinsatz des zurückgewonnenen Kobalts als Hydrocarboxylierungskatalysator :

Die zu Beginn des Beispiels 1 beschriebene Hydrocarboxylierung wird unter den gleichen Bedingungen wiederholt, mit der Ausnahme, daß nun als Katalysator ein in Methanol gelöstes Gemisch aus 63,4 g des violetten, kristallinen Feststoffs und 33 mg Kobaltacetat (zum Ersatz der Kobaltverluste) eingesetzt wird. Die ebenfalls nach 3 Stunden abgebrochene Reaktion liefert das gleiche Ergebnis wie die zu Beginn beschriebene Hydrocarboxylierung.

Beispiel 2

Die in Beispiel 1 beschriebene Hydrocarboxylierung wird wiederholt, mit der Ausnahme, daß als Katalysator für die Hydrocarboxylierung 111,3 g Kobaltnaphthenat mit einem Kobaltgehalt von 15 % eingesetzt werden.

Die oxidative Behandlung des Reaktionsaustrags der Hydrocarboxylierung erfolgt unter den gleichen Bedingungen wie in Beispiel 1.

Als Sumpfprodukt einer stufenweisen destillativen Aufarbeitung des oxidativ vorbehandelten Reaktionsgemisches verbleiben 132,7 g Rückstand mit einem Kobaltgehalt von 12,6 %.

Dieser Rückstand wird auf die gleiche Weise wie in Beispiel 1 durch zweimalige Extraktion aufgearbeitet. Die vereinigten wäßrig-essigsauren Phasen werden wie in Beispiel 1 zur Trockne eingeengt und liefern 64,5 g eines violetten, kristallinen Feststoffs mit einem Kobaltgehalt von 25,8 %. Dies entspricht einer Kobaltrückgewinnung von 99,7 %.

Beispiel 3

Beispiel 1 wird wiederholt, mit der Ausnahme, daß nur 30 % des nach der destillativen Aufarbeitung des oxidativ behandelten Austrags der Hydrocarboxylierung verbleibenden kobalthaltigen Rückstands aufgearbeitet werden. Die Mengen der zur extraktiven Behandlung eingesetzten Essigsäure sowie des Wassers werden entsprechend der geringeren Rückstandsmenge reduziert. Als Endprodukt der Katalysatoraufarbeitung erhält man 19,2 g eines 26,1 % Kobalt enthaltenden violetten, kristallinen Feststoffs. Dies entspricht einer Kobaltrückgewinnung von 99,9 %, bezogen auf den aufgearbeiteten Anteil des kobalthaltigen Rückstands.

19,2 g des violetten, kristallinen Feststoffs und 20 mg Kobaltacetat (zum Ersatz der Kobaltverluste) werden in Methanol gelöst und zusammen mit den 70 % nichtaufgearbeiteten Kobaltrückstands als Hydrocarboxylierungskatalysator eingesetzt. Dabei werden die Bedingungen der in Beispiel 1 zu Beginn beschriebenen Hydrocarboxylierung angewandt. Nach 3 stündiger Reaktion beträgt der Olefinumsatz 86 %.

Beispiel 4

Beispiel 1 wird wiederholt, jedoch wird der als der Sumpf der destillativen Aufarbeitung verbleibende kobalthaltige Rückstand vor einer Aufarbeitung mit 200 ml n-Hexan versetzt.

Der Grad der so erzielbaren Kobaltrückgewinnung beträgt 99,97 %.

Beispiel 5

Beispiel 1 wird wiederholt, jedoch wird anstelle der zur extraktiven Aufarbeitung eingesetzten Essigsäure jeweils die gleiche Gewichtsmenge Propionsäure verwendet.

Als Endprodukt der Katalysatoraufarbeitung erhält man ein violettes, pastöses Produkt, das sich in Alkanolen, wie z. B. Methanol und Ethanol, sowie in γ-Picolin löst und 99,85 % des als Hydrocarboxylierungskatalysator eingesetzten Kobalts enthält.

Beispiel 6

Beispiel 1 1 wird wiederholt, mit der Ausnahme, daß zur extraktiven Katalysatoraufarbeitung nur 50 % der in Beispiel 1 angegebenen Wassermenge eingesetzt wird.

Der Grad der so erzielbaren Kobaltrückgewinnung beträgt 99,85 %.

Beispiel 7

Beispiel 1 wird wiederholt, jedoch wird anstelle von 800 g in der Hydrocarboxylierung eingesetzten Methanols die gleiche Gewichtsmenge an Ethanol verwendet.

Um den als Endprodukt der Katalysatoraufarbeitung verbleibenden violetten, kristallinen Feststoff, der 99,9 % des als Hydrocarboxylierungskatalysator eingesetzten Kobalts enthält, wieder als Lösung in einem der für die Hydrocarboxylierung verwendeten Reaktanten in die Reaktion zurückführen zu können, wird er in ein anderes carbonsaures Salz umgewandelt : 64,2 g des violetten, kristallinen Feststoffs, der sich in keinem der Reaktanten ausreichend löst, werden mit 150,3 g 2-Ethylhexansäure versetzt und solange im Wasserstrahlvolumen erhitzt, bis keine Essigsäure und kein Wasser mehr abdestillieren.

Das so erhaltene Kobaltoctoat wird nach Ersatz der Kobaltverluste als ethanolische Lösung erneut als Katalysator für die Hydrocarboxylierung eingesetzt. Dabei werden die gleichen Bedingungen wie bei der zu Beginn des Beispiels 7 beschriebenen Hydrocarboxylierung eingehalten. Das Reaktionsergebnis beider Hydrocarboxylierungsansätze des Beispiels 7 unterscheidet sich nicht.

Beispiel 8

Beispiel 1 wird wiederholt, mit der Ausnahme, daß anstelle von Dodecen 1 960 g α-Tetradecen eingesetzt werden.

Der Grad der so erzielbaren Kobaltrückgewinnung beträgt 99,88 %.

**Ansprüche**

1. Verfahren zur Aufarbeitung eines kobalthaltigen Destillationssumpfes, der bei der destillativen Aufarbeitung eines durch Umsetzung von Olefinen mit Kohlenmonoxid und Wasser oder Alkanolen in Gegenwart eines kobalthaltigen Katalysators und eines Promotors erhaltenen und anschließend oxidativ behandelten Reaktionsgemisches anfällt, dadurch gekennzeichnet, daß man den als Destillationssumpf anfallenden kobalthaltigen Rückstand mit einer 1 bis 4 Kohlenstoffatome enthaltenden Carbonsäure und Wasser behandelt, die entstehenden Phasen trennt, aus der Carbonsäure-Wasser-Phase das Kobalt in Form des entsprechenden carbonsauren Salzes zurückgewinnt und es gegebenenfalls in ein anderes carbonsaures Salz umwandelt.

Ersetzt durch Anspruch 1, eingegangen am 25.9.82.

2. Verfahren nach Anspruch 1, dadurch gekennzeichnet, daß man als Carbonsäure Essigsäure oder Propionsäure einsetzt.

**Claims**

1. A process for working-up a cobalt-containing distillation bottoms product which has arisen in the working-up by distillation of a reaction mixture obtained by reaction of an olefin with carbon monoxide and water or an alkanol in the presence of a cobalt-containing catalyst and a promoter and subsequently oxidised, characterised in that the cobalt-containing residue arising as distillation bottoms product is treated with a carboxylic acid of 1 to 4 carbon atoms and water, the resulting phases are separated, the cobalt is recovered from the carboxylic acid/water phase in the form of the corresponding carboxylate and that salt is optionally converted into another carboxylate.

2. A process according to claim 1, characterised in that acetic acid or propionic acid is used as the carboxylic acid.

**Revendications**

1. Procédé de traitement d'une queue de distillation qui renferme du cobalt et se forme lorsqu'on traite par distillation un mélange réactionnel obtenu en faisant réagir des oléfines sur du monoxyde de carbone et de l'eau ou des alcanols en présence d'un catalyseur renfermant du cobalt et d'un promoteur, puis en traitant ensuite par oxydation, ledit procédé étant caractérisé par le fait que l'on traite, par un acide carboxylique renfermant de un à 4 atomes de carbone, et par de l'eau, le résidu renfermant du cobalt, que l'on sépare les phases qui se forment, qu'on récupère, à partir de la phase acide carboxylique-eau, le cobalt sous la forme du carboxylate correspondant et qu'on le transforme éventuellement en un autre carboxylate.

2. Procédé selon la revendication 1, caractérisé par le fait que l'on utilise, comme acide carboxylique, l'acide acétique ou l'acide propionique.